# EUROPEAN PATENT SPECIFICATION

(11) **EP 4 488 800 B1**
(45) Date of publication and mention of the grant of the patent: **26.08.2026**
(21) Application number: 23183665.1
(22) Date of filing: 05.07.2023
(51) Int. Cl.: G06F 3/01, A61B 5/00, G09B 19/00

(54) **HUMAN POSE ESTIMATION**
SCHÄTZUNG DER MENSCHLICHEN HALTUNG
ESTIMATION DE POSE HUMAINE

(43) Date of publication of application: 08.01.2025
(73) Proprietor: Hitachi, Ltd., Tokyo 100-8280 (JP)
(72) Inventor: AKIYAMA, Takayuki, 85445 Oberding (DE)
(74) Representative: Mewburn Ellis LLP

(56) References cited:
- CN-A- 116 108 391
- KR-A- 20220 061 526
- US-A1- 2022 304 593

## Description

### TECHNICAL FIELD

The present disclosure relates to a system, computer-implemented method, non-transitory computer-readable storage medium, and computer for human pose estimation.

### BACKGROUND

In managing health and safety of workers, active monitoring technology has been deployed so as to know the current condition of a worker. Motion capture, using smart clothing where inertial measurement unit (IMU) sensors are attached to normal clothing, is emerging as a promising technology as it does not disturb the workers and can provide continuous results. However, there can be large errors in the data collected during motion capture if the item of clothing is incorrectly worn e.g., the sleeve is twisted.

It would be advantageous then to know whether the worker is correctly wearing the item of clothing. CN107744393A proposes a gravity acceleration sensor-based wearable for use in a posture monitoring system. The gravity acceleration sensors are mounted to the back of the clothes, and changes to the operator's posture is sensed together with respiratory parameters.

US 2022/304593 A1 proposes a posture recognition system that can properly respond to an error that is changed according to a user's motion. A posture estimation section calculates posture data indicating a posture of a user wearing clothing, on the basis of motion data measured by a posture sensor attached to the clothing. An error estimation section calculates error data which is an estimate of an error that is occurring in the posture data, on the basis of the motion data and the posture data.

Further background art can be found in KR 2022 0061526 A and CN 116 108 391 A.

The present disclosure was arrived at in light of the above considerations.

### SUMMARY

Accordingly, in a first aspect, embodiments of the present invention provide a system as set out in claim 1.

Such a system can quickly and accurately estimate a pose of the operator and can mitigate against errors which would arise from the operator wearing the item of clothing incorrectly.

The system may include any one, or any combination insofar as they are compatible, of the optional features set out with reference to the first aspect.

The sensor(s) attached to the item of clothing may be inertial measurement units (IMUs).

The first estimate of the pose and the second estimate of the pose defines the position and orientation of one or more joints of the operator. For the avoidance of doubt, the first and/or second estimate of the pose may, but does not necessarily have to be, an entire pose of the operator, e.g., it may include only a partial pose of the operator. For example, the first and/or second estimate of the pose may only define the position and/or orientation of a single joint of the operator (e.g., wrist joint). By distance it may be meant an angular distance, for example a first vector describing the projection of a portion of the body according to the first estimate as compared to second vector describing the projection of the same portion of the body according to the second estimate.

The optical sensor may be a camera. The camera may be a security camera, for example fixed to a portion of infrastructure. The camera may be a wearable head-mounted camera. The processor may be configured to detect the operator's hand(s) using data from the wearable head-mounted camera and utilise the detected position of the operator's hand(s) in the second estimate of the pose of the operator.

The processor may be configured to identify when the operator is within a capture area of the optical sensor, and to trigger the optical sensor to capture the second estimate of the pose when it has been identified that the operator is within the capture area. When the camera is a wearable head-mounted camera, it may be worn by a second operator different to the operator wearing the item of clothing (and so detection may occur when this second operator directs the camera to the operator wearing the item of clothing).

The processor may be configured to alert the operator when it is determined that the operator is wearing the item of clothing incorrectly.

The first estimate and second estimate each respectively include data defining a position and orientation of one or more joints of the operator's body. Calculating the difference includes calculating a distance between a joint a defined by the first estimate and the same joint as defined by the second estimate. The processor is configured to determine that the operator is wearing the item of clothing incorrectly by determining a similarity between the calculated distance for each joint and a stored distance in an error database, and the processor determines that the operator is wearing the item of clothing incorrectly when the determined similarity is greater than a threshold.

The system may further comprise an augmented reality device, and the processor may be configured, in response to determining that the operator is wearing the item of clothing incorrectly, to provide guidance to the operator via the augmented reality device. The augmented reality device may be, for example, a virtual reality headset, augmented reality headset, or mixed reality headset.

The processor may be configured, in response to determining that the operator is wearing the item of clothing incorrectly, to detect a dangerous action being performed by the operator.

In a second aspect, embodiments of the present invention provide a computer-implemented method as set out in claim 9.

In a third aspect, embodiments of the present invention provide a computer as set out in claim 11.

The computer may include an optical pose estimation module, motion capturing module, and error detection module.

In a fourth aspect, embodiments of the present invention provide a non-transitory computer-readable storage medium containing machine-executable instructions which, when executed on a processor, cause the processor to perform the method of the second aspect.

The invention includes the combination of the aspects and preferred features described except where such a combination is clearly impermissible or expressly avoided.

Further aspects of the present invention provide: a computer program comprising code which, when run on a computer, causes the computer to perform the method of the second aspect; a computer readable medium storing a computer program comprising code which, when run on a computer, causes the computer to perform the method of the second aspect; and a computer system programmed to perform the method of the second aspect.

### BRIEF DESCRIPTION OF THE DRAWINGS

Figure 1 shows a system;
Figs. 2A and 2B illustrate a first and second estimated pose;
Figure 3 is a flow chart illustrating a method;
Figure 4 is a table showing examples of joint distance thresholds used in determining incorrect wearing;
Figure 5 shows a variant system;
Figure 6 shows a method of using the variant system of Figure 5;
Figure 7 shows a variant system;
Figure 8 shows a method of using the variant system of Figure 7;
Figure 9 shows the use of hand position for identifying incorrect wearing;
Figure 10 shows a variant system;
Figure 11 shows a variant system; and
Figure 12 shows a variant system.

### DETAILED DESCRIPTION

Aspects and embodiments of the present invention will now be discussed with reference to the accompanying figures. Further aspects and embodiments will be apparent to those skilled in the art.

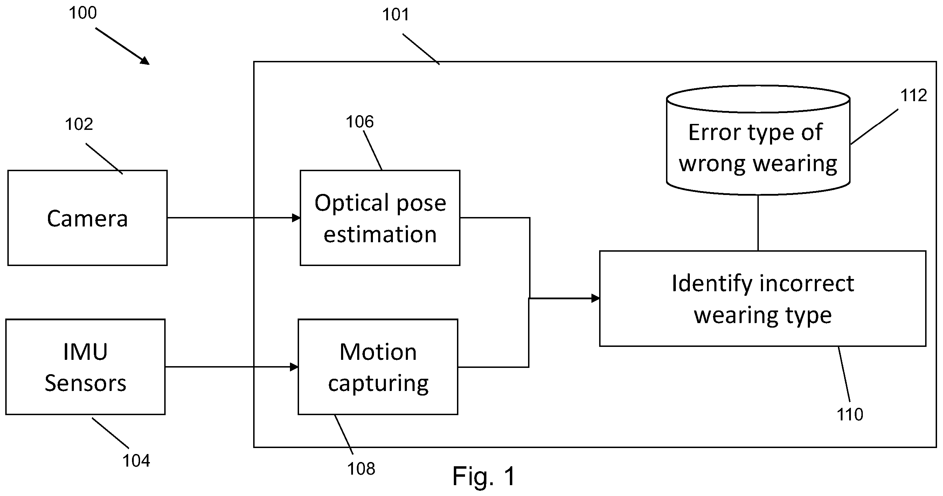

Figure 1 shows a system 100, which includes a processor 101 connected to a camera 102 and one or more IMU sensors attached to an item of clothing 104. The processor contains or operates an optical pose estimation module 106 and a motion capturing module 108, both of which may be implemented as pieces of software. In some examples, the processor is a distributed processor (e.g., comprises one or more processors connected together).

The motion capturing module 108 receives data from one or more IMU sensors 104 and utilises this in obtaining a first estimate of a pose of an operator wearing the item of clothing. The motion capturing module 108 uses methods known *per se* in the art (e.g. Inertial-Based Human Motion Capture: A Technology Summary of Current Processing Methodologies for Spatiotemporal and Kinematic Measures, Hindle et al., Appl. Bionics Biomech. 2021; 6628320). One or more signal from the one or more sensors which are attached to a body of the person are received by the motion capturing module 108. Based on these signals, skeletal data is created. The IMU sensors may include, for example, at least one accelerometer, at least one gyroscope, and at least one magnetometer. The skeletal data may be created by using the sensor signals to model a kinematic chain, which can describe the motion constraints of an articulated rigid body such as the human skeleton. The concept underpinning a kinematic chain is that the motion of a body segment is given by the motion of the previous body segment in the chain and an angular rotation around a joint axis. The kinematic chain can therefore be defined with a six degrees of freedom root joint representing the global rigid body motion and a set of one degree of freedom revolute joints describing the angular motion of the limbs. The created skeletal data may include position and/or orientation of parts of the person's body.

The optical pose estimation module receives data from the camera and utilises this in obtaining a second estimate of a pose of the operator wearing the item of clothing. The optical pose estimation module can use methods known per se in the art (see, e.g., the OpenPose library from Viso Suite). The first and second estimated poses are provided to an error detecting module 110, which uses the first and second estimated poses together with a database 112 to identify if the operator is wearing the item of clothing incorrectly. It also determines a type of wrong wearing, for example that the sleeve of the item of clothing is flipped or twisted around the forearm.

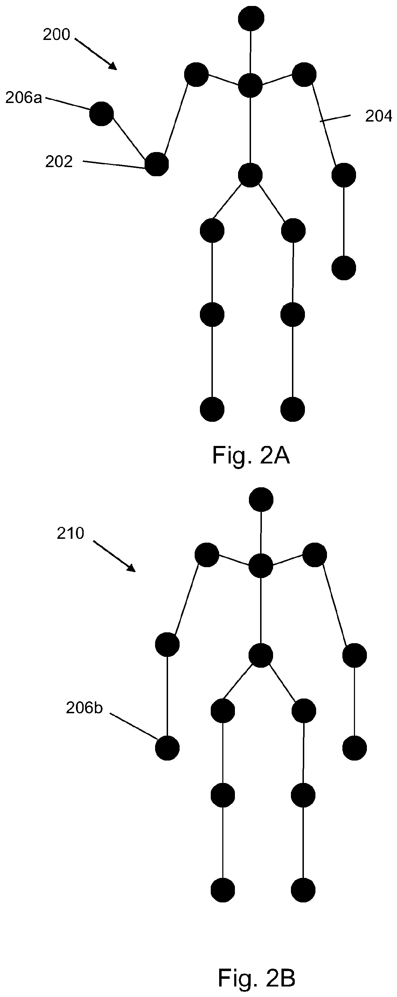

Figs. 2A and 2B illustrate a first 200 and second 210 estimated pose. Each pose is formed of joints 202 of the body connected by links 204, for example hand joint 206a,b. In the first 200 estimated pose is obtained from the IMU sensor data, but as the right sleeve of the item of clothing was flipped it shows that a hand joint 206a of the operator is located above the elbow joint 202 of the operator. Whereas the hand joint 206b in the second 210 estimated pose is located below the elbow joint 202 of the operator, illustrating a difference between the first and second estimated poses.

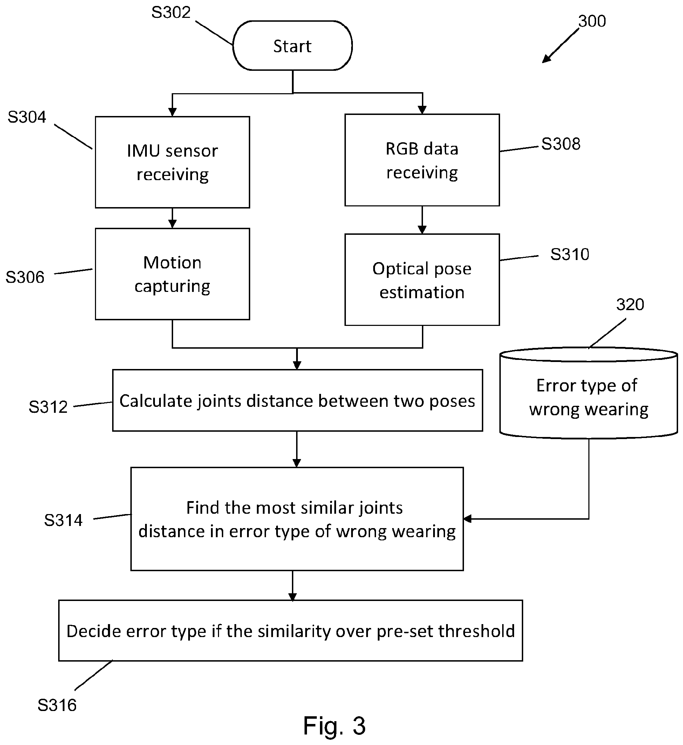

Figure 3 is a flow chart illustrating a method 300. The method begins with step S302, and proceeds down two parallel paths. In step S304, IMU sensor data is received from the one or more IMU sensors attached to the item of clothing worn by the operator. Next, in step S306, this IMU data is used in a motion capture step S306 to derive a first estimate of the pose of the operator.

At the same time, before, or in parallel to steps S304 and S306, in step S308 video data (e.g., RGB data) is received from an optical sensor. This RGB data is indicative of a second estimate of a pose of the operator by virtue of capturing an image of the operator. Next, in step S310, an optical pose estimation step is performed using the received RGB data to derive a second estimate of the pose of the operator.

After steps S306 and S310 have been completed, the method moves to step S312 where joint distances are calculated between the two poses. That is, a distance is calculated between a joint as defined in the first estimate and the same joint as defined in the second estimate. For example, the distance between the position of the operator's left elbow as defined in the first estimate and the position of the operator's left elbow as defined in the second estimate is calculated.

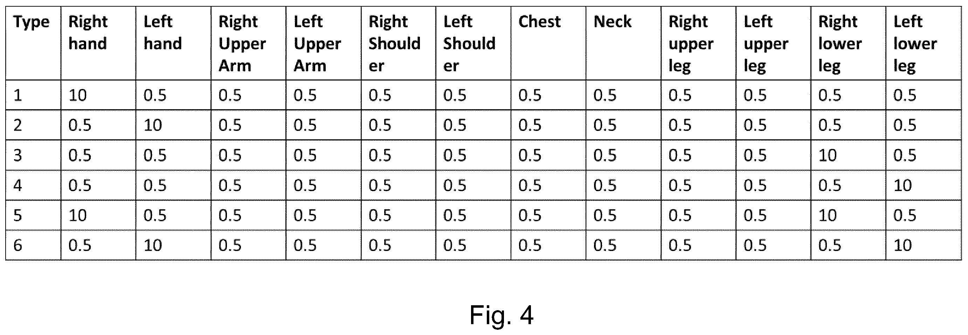

Once the calculations have been performed for all joints considered, the method moves to step S314 where reference is made to a database 320 to find the most similar joints distance in the database 320. The database contains a table where distances between joints is related to an incorrect wearing type. An example of such a table is shown in Figure 4. The type (e.g., right hand error) is identified which has values in the database which are most similar to the observed distances on joints between the IMU motion capture and the optical pose. The method moves to step S316, where an error type of wearing the item of clothing is decided if a given similarity is over a pre-set threshold.

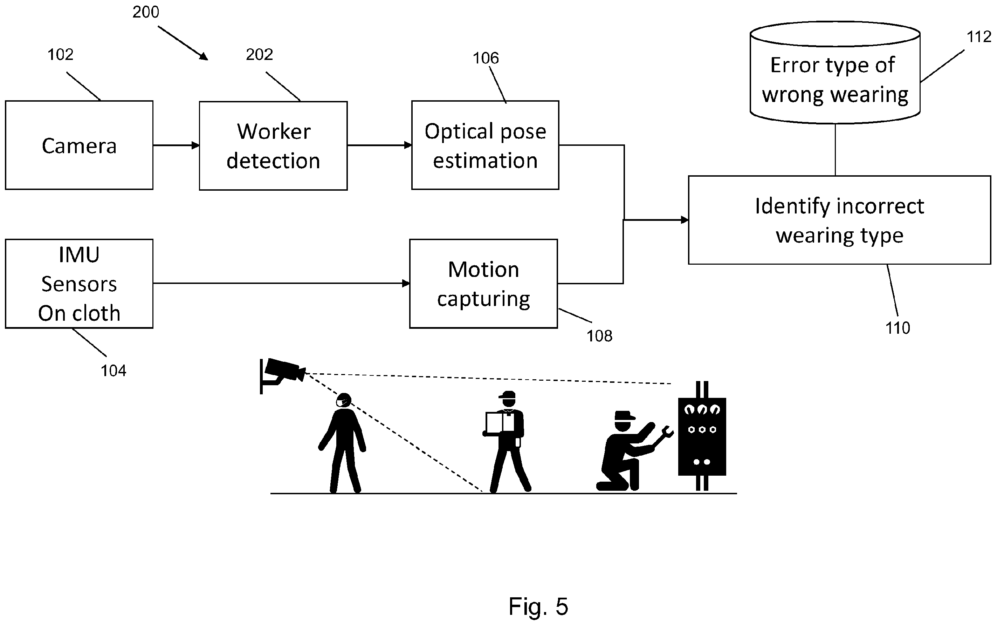

Figure 5 shows a variant system 200. Where the system 200 includes features in common with system 100 in Figure 1, like features are indicated by like reference numerals. In contrast to system 100 in Figure 1, system 200 includes a worker detection module 202. The worker detection module may be configured to utilise the data received from camera 102 to trigger the optical pose estimation module 106 when it detects the presence of the worker wearing the item of clothing with the IMU sensor(s) attached. Further, where there are multiple people within the image captured by the camera 102, the worker detection module 202 may be utilised to identify which of the people is the one wearing the item of clothing with the IMU sensors attached. For example, all people within the image captured by the camera may be optically tracked and then one who's movements correspond to the motion captured by the IMU is identified. In some examples, the clothing may include an optical marker such that the system can identify the user wearing the item of clothing through detecting this optical marker.

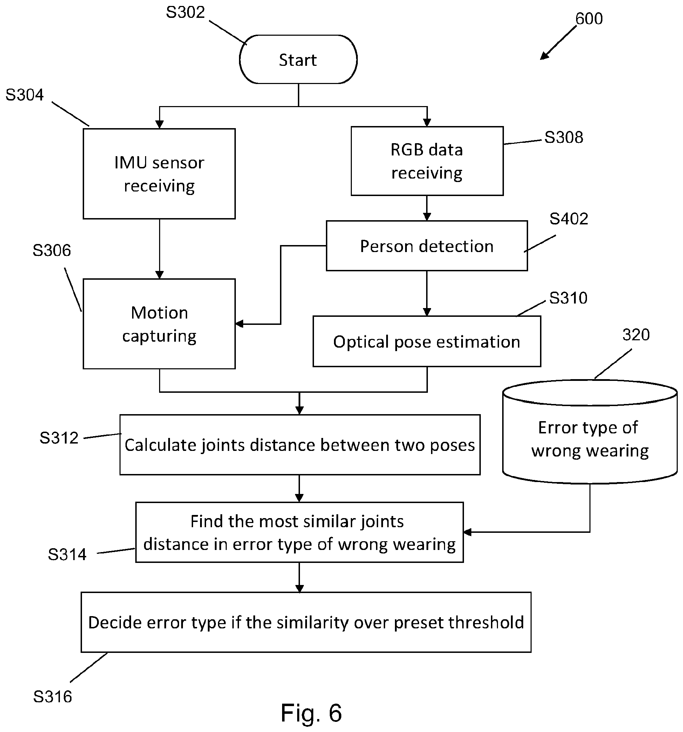

Figure 6 shows a method 600 of using the variant system of Figure 5. Where it shares features with the method 300 shown in Figure 3, like features are indicated by like reference numerals. In contrast to the method 300 shown in Figure 3, method 600 includes an additional step S402 located between steps S308 and S310. The RGB data is received in step S308, and then a person detection step is performed in S402. As discussed with respect to Figure 5, in the person detecting step, the operator wearing the item of clothing may be detected from the captured RGB data received from the camera 308. This may trigger the motion capturing step S306 to derive the first estimate of the pose of the operator. The person detection step S402 may also include identifying which person, of multiple people within view of the camera, is wearing the item of clothing with the IMU sensor(s) attached thereto.

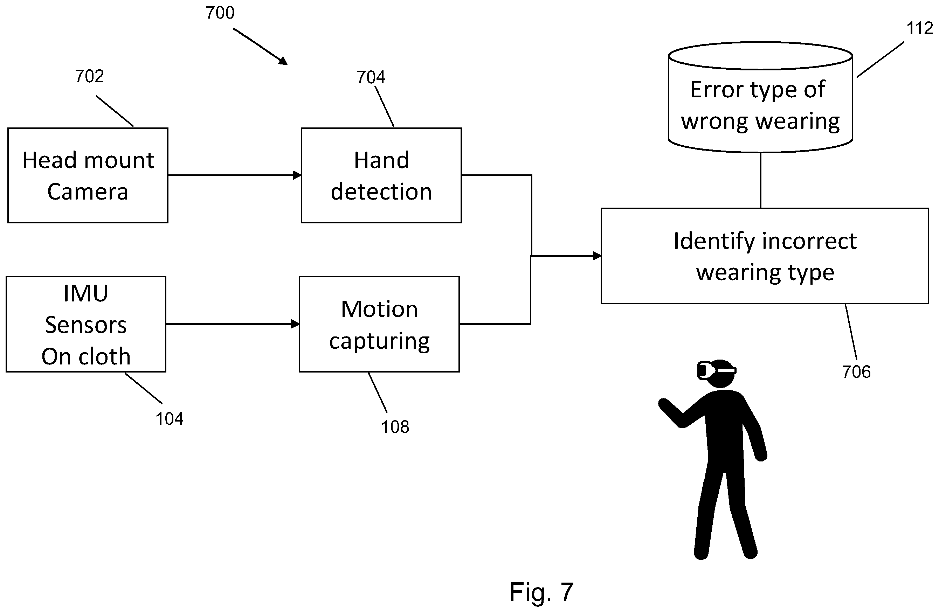

Figure 7 shows a variant system 700. Where the system 700 includes features in common with the system 100 in Figure 1, like features are indicated by like reference numerals. In contrast to the system 100 in Figure 1, the camera in system 700 is a head-mounted camera (in Figures 1 and 5, the camera is a static camera for example a security camera). The system 700 further includes a hand detection module 704, which utilises the data captured from the head mounted camera 702 to determine the second estimate of the pose of the operator (e.g., the position of the operator's hand(s)). A variant error detecting module 706 utilises the database 112 together with the first estimate from motion capturing module 108 and the second estimate from the hand detection module 704 to determine if the operator is wearing the item of clothing incorrectly.

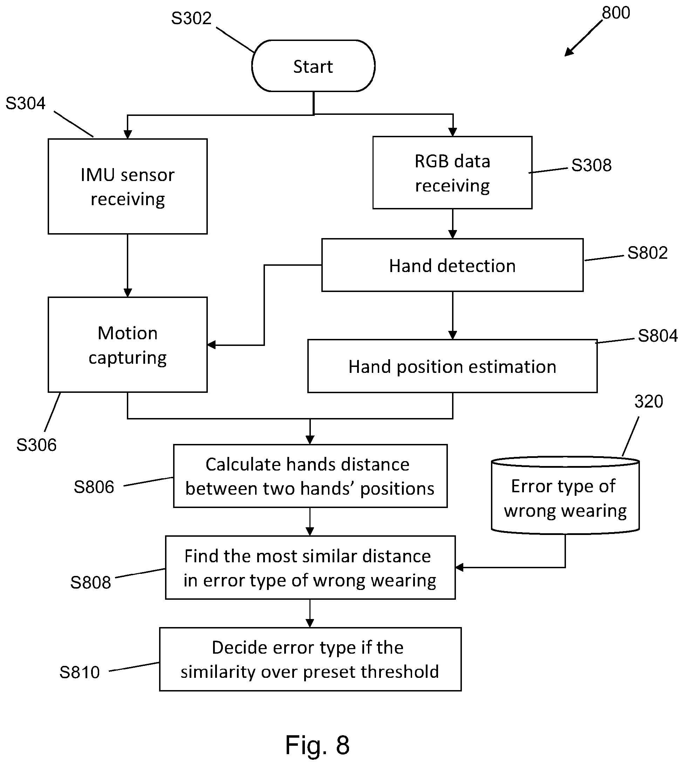

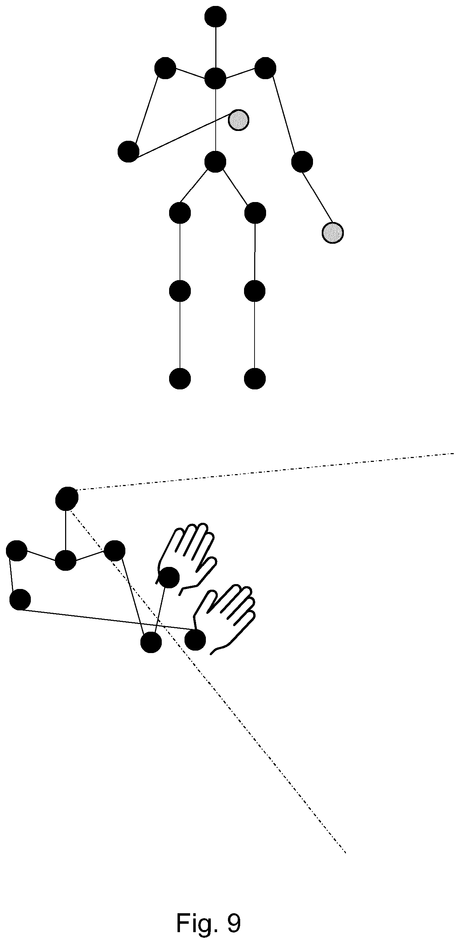

Figure 8 shows a method 800 of using the variant system of Figure 7. Where the method 700 shares features with the methods 300 and 600 shown in Figures 3 and 6, like features are indicated by like reference numerals. The method differs in that step S308 is followed by step S802 in which a hand detection step is performed. The hand detection step utilises the RGB data received from the head mounted camera, and when the operator's hand(s) are detected from that data the motion capturing step S306 can be triggered. A hand position estimation step S804 is performed, using the RGB data, to derive the second estimate of the operator's pose. The distance between the position of the operator's hand(s) as defined in the first and second estimates respectively is then calculated in step S806. Once the calculations have been performed for all joints considered, the method moves to step S808 where reference is made to a database 320. The database contains a table where distances between joints is related to an incorrect wearing type. An example of such a table is shown in Figure 4. The method moves to step S810, where an error type of wearing the item of clothing is decided if a given similarity is over a pre-set threshold. Figure 9 shows the use of hand position for identifying incorrect wearing. In such an example, the step S314 in Figure 3 is modified so that the distance is calculated only for the hands as detected by the IMU and optically tracked by the head mounted camera.

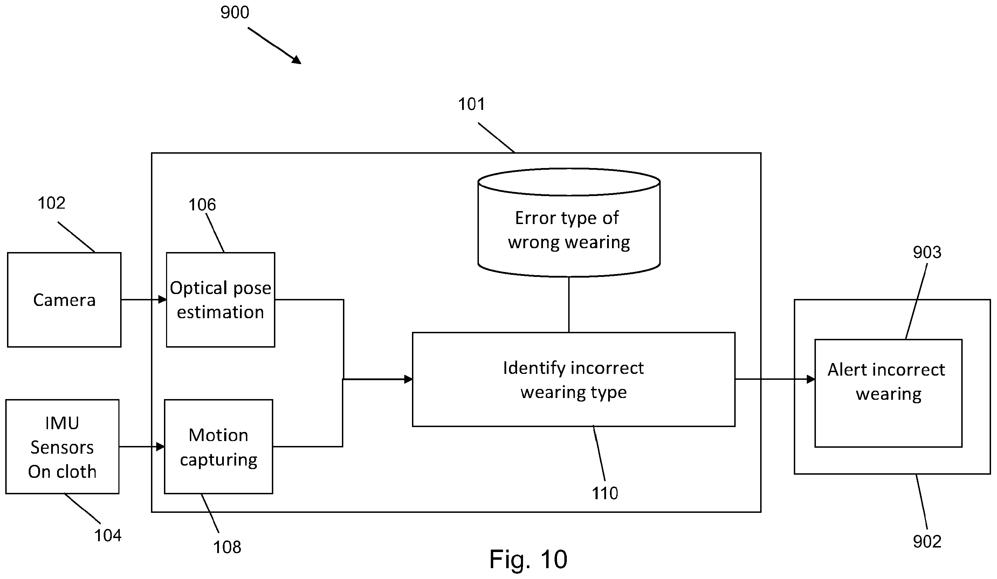

Figure 10 shows a variant system 900. Where the system 900 shares features with system 100 in Figure 1, like features are indicated by like reference numerals. The system 900 differs from system 100 in that it further includes an operator user interface module 902 (e.g., headphones, display, speakers, or haptic feedback device). The operator user interface module 902 includes an alert module 903, which receives an indication from processor 101 that the operator is incorrectly wearing the item of clothing. In response, the alert module 903 notifies the operator. The notification may notify that the item of clothing is being incorrectly worn with or without an indication as to the type of incorrect wearing (e.g., twisted sleeve).

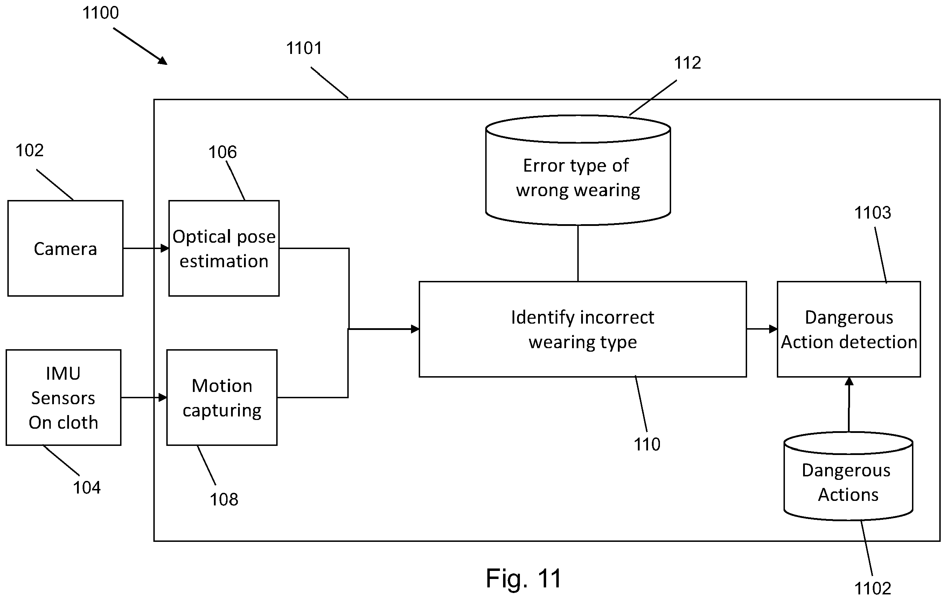

Figure 11 shows a variant system 1100. Where the system 1100 shares features with system 100 in Figure 1, like features are indicated by like reference numerals. The system 1100 differs in that processor 1101 also includes a dangerous action detection module 1103 which utilises a dangerous actions database 1102 to detect a dangerous human action (e.g., one which may lead to a dangerous situation for the person carrying out the working process or other persons in the vicinity).

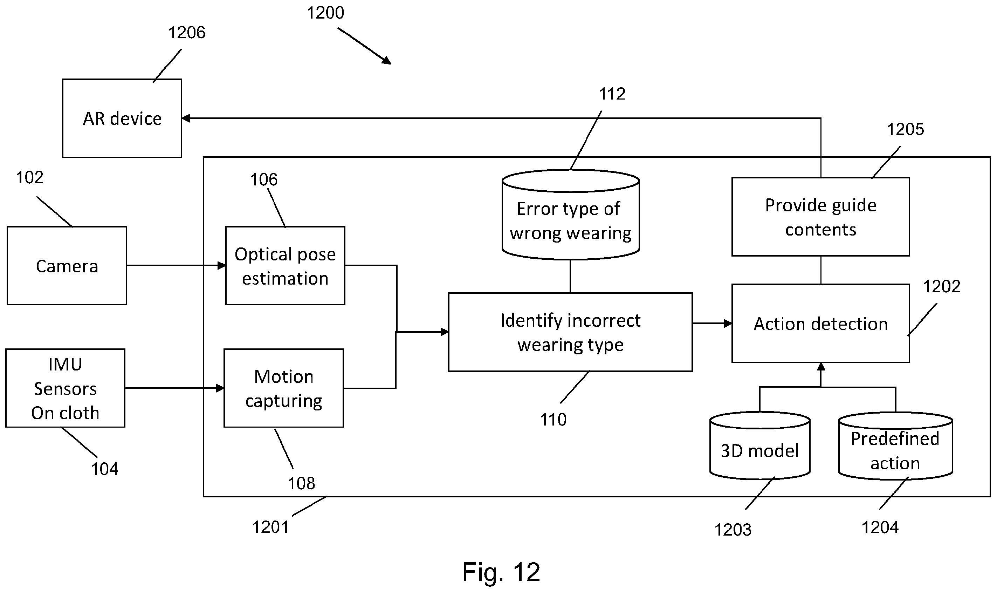

Figure 12 shows a variant system 1200. Where the system 1200 shares features with system 100, like features are indicated by like reference numerals. The system 1200 differs in that the processor 1201 also includes an action detection module 1202 which utilises data from the error detecting module 110, a 3D model 1203, and a predefined action database 1204 to detect an action being or about to be performed by the operator. Guidance module 1205 receives data from the action detection module 1202, and outputs guidance to an AR device 1206 worn by the operator. For example, the system may detect a dangerous action of the person by comparing the person's estimated pose with the plurality of dangerous actions stored in the dangerous action database. If a dangerous action is determined by said comparison, a warning signal can be generated by a warning device can be outputted via an output device. The warning signal may be a visual, audible, and/or haptic warning signal that alerts the operator that their current action may lead to a dangerous situation.

The guidance process may include the predefined action database storing a plurality of predetermined working actions. A working action in this sense may be a human action related to a working process, such as assembling, drilling, jiggering, etc. The guidance database may include information for supporting, improving, and/or optimizing a working process as well as information for avoiding a dangerous situation throughout the working process. The AR device may be, for example, a pair of glasses, a smartphone, a tablet, or any other device suitable for providing an augmented reality interface.

The system may detect a working action of the person by comparing the person's estimated poses with a plurality of working actions stored on the working action database. Guidance information may be determined related to the detected working action based on the plurality of guidance information stored in the guidance database. Subsequently, the system may output the determined guidance information to the augmented reality device work and/or carried by the person via the output device (not shown).

The systems and methods of the above embodiments may be implemented in a computer system (in particular in computer hardware or in computer software) in addition to the structural components and user interactions described.

The term "computer system" includes the hardware, software and data storage devices for embodying a system or carrying out a method according to the above-described embodiments. For example, a computer system may comprise a central processing unit (CPU), input means, output means and data storage. The computer system may have a monitor to provide a visual output display. The data storage may comprise RAM, disk drives or other computer readable media. The computer system may include a plurality of computing devices connected by a network and able to communicate with each other over that network.

The methods of the above embodiments may be provided as computer programs or as computer program products or computer readable media carrying a computer program which is arranged, when run on a computer, to perform the method(s) described above.

The term "computer readable media" includes, without limitation, any non-transitory medium or media which can be read and accessed directly by a computer or computer system. The media can include, but are not limited to, magnetic storage media such as floppy discs, hard disc storage media and magnetic tape; optical storage media such as optical discs or CD-OMs; electrical storage media such as memory, including RAM, ROM and flash memory; and hybrids and combinations of the above such as magnetic/optical storage media.

While the disclosure has been described in conjunction with the exemplary embodiments described above, many equivalent modifications and variations will be apparent to those skilled in the art when given this disclosure. Accordingly, the exemplary embodiments of the disclosure set forth above are considered to be illustrative and not limiting. Various changes to the described embodiments may be made.

While the disclosure has been described in conjunction with the exemplary embodiments described above, many equivalent modifications and variations will be apparent to those skilled in the art when given this disclosure. Accordingly, the exemplary embodiments of the disclosure set forth above are considered to be illustrative and not limiting. Various changes to the described embodiments may be made.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means for example +/- 10%.

## Claims

1. A system (100) for performing human pose estimation, the system comprising:
an item of clothing including one or more sensors (104) attached thereto, the or each sensor being configured to obtain data indicative of a first estimate of a pose of an operator wearing the item of clothing;
an optical sensor (102, 702), configured to obtain data indicative of a second estimate of the pose of the operator; and
a processor (101), configured to:
calculate a difference between the first estimate of the pose and the second estimate of the pose,
determine, based on the calculated difference, whether the operator is wearing the item of clothing incorrectly, and
alert the operator when it is determined that the operator is wearing the item of clothing incorrectly;
wherein the first estimate and the second estimate each respectively include data defining a position and orientation of one or more joints of the operator's body;
wherein calculating the difference includes calculating a distance between a joint as defined in the first estimate and the same joint as defined in the second estimate; and
wherein the processor is configured to determine that the operator is wearing the item of clothing incorrectly by determining a similarity between the calculated distance for each joint and a stored distance in an error database (112, 320), and wherein the processor determines that the operator is wearing the item of clothing incorrectly when the determined similarity is greater than a threshold.

2. The system of claim 1, wherein the sensor(s) attached to the item of clothing are inertial measurement units.

3. The system of claim 1 or 2, wherein the optical sensor is a camera.

4. The system of claim 3, wherein the camera is a wearable head-mounted camera.

5. The system of claim 4, wherein the processor is configured to detect the operator's hand(s) using data from the wearable head-mounted camera, and utilise the detected position of the operator's hand(s) in the second estimate of the pose of the operator.

6. The system of any preceding claim, wherein the processor is configured to identify when the operator is within a capture area of the optical sensor, and to trigger the optical sensor to obtain the data indicative of the second estimate of the pose when it has been identified that the operator is within the capture area.

7. The system of any preceding claim, further comprising an augmented reality device, wherein the processor is configured, in response to determining that the operator is wearing the item of clothing incorrectly, to provide guidance to the operator via the augmented reality device.

8. The system of any preceding claim, wherein the processor is configured, in response to determining that the operator is wearing the item of clothing incorrectly, to detect a dangerous action being performed by the operator by utilising a dangerous actions database to detect the dangerous human action.

9. A computer-implemented method (800) for performing human pose estimation, the method comprising steps of:
receiving (S304), from one or more sensors attached to an item of clothing, data indicative of a first estimate of a pose of an operator wearing the item of clothing;
receiving (S308), from an optical sensor, data indicative of a second estimate of the pose of the operator;
calculating (S312) a difference between the first estimate of the pose and the second estimate of the pose;
determining, based on the calculated difference, whether the operator is wearing the item of clothing incorrectly; and
alert the operator when it is determined that the operator is wearing the item of clothing incorrectly;
wherein the first estimate and the second estimate each respectively include data defining a position and orientation of one or more joints of the operator's body;
wherein calculating the difference includes calculating a distance (S312) between a joint as defined in the first estimate and the same joint as defined in the second estimate; and
wherein the processor is configured to determine that the operator is wearing the item of clothing incorrectly by determining (S314) a similarity between the calculated distance for each joint and a stored distance in an error database, and wherein the processor determines that the operator is wearing the item of clothing incorrectly when the determined (S316) similarity is greater than a threshold.

10. A non-transitory computer-readable storage medium containing machine-executable instructions which, when executed on a processor, cause the processor to perform the method of claim 9.

11. A computer comprising a processor and memory, the memory containing machine-executable instructions which, when executed on the processor, cause the processor to:
receive, from one or more sensors attached to an item of clothing, data indicative of a first estimate of a pose of an operator wearing the item of clothing:
receive, from an optical sensor, data indicative of a second estimate of the pose of the operator;
calculate a difference between the first estimate of the pose and the second estimate of the pose;
determine, based on the calculated difference, whether the operator is wearing the item of clothing incorrectly; and
alert the operator when it is determined that the operator is wearing the item of clothing incorrectly;
wherein the first estimate and the second estimate each respectively include data defining a position and orientation of one or more joints of the operator's body;
wherein calculating the difference includes calculating a distance between a joint as defined in the first estimate and the same joint as defined in the second estimate; and
wherein the processor is configured to determine that the operator is wearing the item of clothing incorrectly by determining a similarity between the calculated distance for each joint and a stored distance in an error database, and wherein the processor determines that the operator is wearing the item of clothing incorrectly when the determined similarity is greater than a threshold.

## Patentansprüche

1. System (100) zum Durchführen einer Schätzung einer menschlichen Haltung, wobei das System Folgendes umfasst:
ein Kleidungsstück, das einen oder mehrere daran befestigte Sensoren (104) umfasst, wobei der oder jeder Sensor dazu ausgelegt ist, Daten zu erhalten, die eine erste Schätzung einer Haltung eines Anwenders, der das Kleidungsstück trägt, angeben;
einen optischen Sensor (102, 702), der dazu ausgelegt ist, Daten zu erhalten, die eine zweite Schätzung der Haltung des Anwenders angeben; und
einen Prozessor (101), der für Folgendes konfiguriert ist:
Berechnen einer Differenz zwischen der ersten Schätzung der Haltung und der zweiten Schätzung der Haltung,
Bestimmen, ob der Anwender das Kleidungsstück korrekt trägt, basierend auf der berechneten Differenz, und
Warnen des Anwenders, wenn bestimmt wird, dass der Anwender das Kleidungsstück nicht korrekt trägt;
wobei die erste Schätzung und die zweite Schätzung jeweils entsprechende Daten umfassen, die eine Position und Ausrichtung von einem oder mehreren Gelenken des Körpers des Anwenders definieren;
wobei das Berechnen der Differenz das Berechnen eines Abstands zwischen einem Gelenk gemäß der Definition der ersten Schätzung und demselben Gelenk gemäß der Definition der zweiten Schätzung umfasst; und
wobei der Prozessor konfiguriert ist, um zu bestimmen, dass der Anwender das Kleidungsstück nicht korrekt trägt, indem er eine Ähnlichkeit zwischen dem berechneten Abstand für jedes Gelenk und einem gespeicherten Abstand in einer Fehlerdatenbank (112, 320) bestimmt, und wobei der Prozessor bestimmt, dass der Anwender das Kleidungsstück nicht korrekt trägt, wenn die bestimmte Ähnlichkeit größer als ein Schwellenwert ist.

2. System nach Anspruch 2, wobei der/die an dem Kleidungsstück befestigte(n) Sensor(en) Trägheitsmesseinheiten sind.

3. System nach Anspruch 1 oder 2, wobei der optische Sensor eine Kamera ist.

4. System nach Anspruch 3, wobei die Kamera eine tragbare, am Kopf montierte Kamera ist.

5. System nach Anspruch 4, wobei der Prozessor konfiguriert ist, um eine Hand/die Hände des Anwenders unter Verwendung von Daten von der tragbaren, am Kopf montierten Kamera zu detektieren und die detektierte Position der Hand/der Hände des Anwenders bei der zweiten Schätzung der Haltung des Anwenders zu verwenden.

6. System nach einem der vorangegangenen Ansprüche, wobei der Prozessor dazu ausgelegt ist, zu identifizieren, wenn der Anwender sich innerhalb eines Erfassungsbereichs des optischen Sensors befindet, und den optischen Sensor auszulösen, um die Daten zu erhalten, welche die zweite Schätzung der Haltung angeben, wenn identifiziert wurde, dass sich der Anwender innerhalb des Erfassungsbereichs befindet.

7. System nach einem der vorangegangenen Ansprüche, ferner umfassend eine Augmented-Reality-Vorrichtung, wobei der Prozessor konfiguriert ist, um als Antwort auf das Bestimmen, dass der Anwender das Kleidungsstück nicht korrekt trägt, für den Anwender über die Augmented-Reality-Vorrichtung eine Anleitung bereitzustellen.

8. System nach einem der vorangegangenen Ansprüche, wobei der Prozessor konfiguriert ist, um als Antwort auf das Bestimmen, dass der Anwender das Kleidungsstück nicht korrekt trägt, eine gefährliche Handlung, die durch den Anwender vorgenommen wird, zu detektieren, indem er eine Datenbank für gefährliche Handlungen verwendet, um die gefährliche menschliche Handlung zu detektieren.

9. Computerimplementiertes Verfahren (800) zum Durchführen einer Schätzung einer menschlichen Haltung, wobei das Verfahren folgende Schritte umfasst:
Empfangen (S304), von einem oder mehreren an einem Kleidungsstück befestigten Sensoren, von Daten, die eine erste Schätzung einer Haltung eines Anwenders, der das Kleidungsstück trägt, angeben;
Empfangen (S308), von einem optischen Sensor, von Daten, die eine zweite Schätzung der Haltung des Anwenders angeben;
Berechnen (S312) einer Differenz zwischen der ersten Schätzung der Haltung und der zweiten Schätzung der Haltung;
Bestimmen, ob der Anwender das Kleidungsstück korrekt trägt, basierend auf der berechneten Differenz; und
Warnen des Anwenders, wenn bestimmt wird, dass der Anwender das Kleidungsstück nicht korrekt trägt;
wobei die erste Schätzung und die zweite Schätzung jeweils entsprechende Daten umfassen, die eine Position und Ausrichtung von einem oder mehreren Gelenken des Körpers des Anwenders definieren;
wobei das Berechnen der Differenz das Berechnen des Abstands (S312) zwischen einem Gelenk gemäß der Definition der ersten Schätzung und demselben Gelenk gemäß der Definition der zweiten Schätzung umfasst; und
wobei der Prozessor konfiguriert ist, um zu bestimmen, dass der Anwender das Kleidungsstück nicht korrekt trägt, indem er eine Ähnlichkeit zwischen dem berechneten Abstand für jedes Gelenk und einem gespeicherten Abstand in einer Fehlerdatenbank bestimmt (S314), und wobei der Prozessor bestimmt, dass der Anwender das Kleidungsstück nicht korrekt trägt, wenn die bestimmte (S316) Ähnlichkeit größer als ein Schwellenwert ist.

10. Nichtflüchtiges computerlesbares Speichermedium, das durch eine Maschine ausführbare Befehle umfasst, die, wenn sie auf einem Prozessor ausgeführt werden, bewirken, dass der Prozessor ein Verfahren nach Anspruch 9 durchführt.

11. Computer, umfassend einen Prozessor und einen Speicher, wobei der Speicher durch eine Maschine ausführbare Befehle umfasst, die, wenn sie auf dem Prozessor ausgeführt werden, bewirken, dass der Prozessor:
von einem oder mehreren an einem Kleidungsstück befestigten Sensoren Daten empfängt, die eine erste Schätzung einer Haltung eines Anwenders, der das Kleidungsstück trägt, anzeigen;
von einem optischen Sensor Daten empfängt, die eine zweite Schätzung der Haltung des Anwenders anzeigen;
eine Differenz zwischen der ersten Schätzung der Haltung und der zweiten Schätzung der Haltung berechnet;
basierend auf der berechneten Differenz bestimmt, ob der Anwender das Kleidungsstück korrekt trägt; und
den Anwender warnt, wenn bestimmt wird, dass der Anwender das Kleidungsstück nicht korrekt trägt;
wobei die erste Schätzung und die zweite Schätzung jeweils entsprechende Daten umfassen, die eine Position und Ausrichtung von einem oder mehreren Gelenken des Körpers des Anwenders definieren;
wobei das Berechnen der Differenz das Berechnen des Abstands zwischen einem Gelenk gemäß der Definition der ersten Schätzung und demselben Gelenk gemäß der Definition der zweiten Schätzung umfasst; und
wobei der Prozessor konfiguriert ist, um zu bestimmen, dass der Anwender das Kleidungsstück nicht korrekt trägt, indem er eine Ähnlichkeit zwischen dem berechneten Abstand für jedes Gelenk und einem gespeicherten Abstand in einer Fehlerdatenbank bestimmt, und wobei der Prozessor bestimmt, dass der Anwender das Kleidungsstück nicht korrekt trägt, wenn die bestimmte Ähnlichkeit größer als ein Schwellenwert ist.

## Revendications

1. Système (100) pour effectuer une estimation de pose humaine, le système comprenant :
un article vestimentaire incluant un ou plusieurs capteurs (104) fixés à celui-ci, le ou chaque capteur étant configuré pour obtenir des données indicatives d'une première estimation d'une pose d'un opérateur portant l'article vestimentaire ;
un capteur optique (102, 702), configuré pour obtenir des données indicatives d'une seconde estimation de la pose de l'opérateur ; et
un processeur (101), configuré pour :
calculer une différence entre la première estimation de la pose et la seconde estimation de la pose,
déterminer, sur la base de la différence calculée, si l'opérateur porte l'article vestimentaire de manière incorrecte, et
alerter l'opérateur lorsqu'il est déterminé que l'opérateur porte l'article vestimentaire de manière incorrecte ;
dans lequel la première estimation et la seconde estimation incluent chacune respectivement des données définissant une position et une orientation d'une ou plusieurs articulations du corps de l'opérateur ;
dans lequel le calcul de la différence inclut le calcul d'une distance entre une articulation telle que définie dans la première estimation et la même articulation telle que définie dans la seconde estimation ; et
dans lequel le processeur est configuré pour déterminer que l'opérateur porte l'article vestimentaire de manière incorrecte en déterminant une similitude entre la distance calculée pour chaque articulation et une distance stockée dans une base de données d'erreurs (112, 320), et dans lequel le processeur détermine que l'opérateur porte l'article vestimentaire de manière incorrecte lorsque la similitude déterminée est supérieure à un seuil.

2. Système selon la revendication 1, dans lequel le ou les capteurs fixés à l'article vestimentaire sont des unités de mesure inertielle.

3. Système selon la revendication 1 ou 2, dans lequel le capteur optique est une caméra.

4. Système selon la revendication 3, dans lequel la caméra est une caméra portable montée sur la tête.

5. Système selon la revendication 4, dans lequel le processeur est configuré pour détecter la ou les mains de l'opérateur en utilisant des données provenant de la caméra portable montée sur la tête, et utiliser la position détectée de la ou des mains de l'opérateur dans la seconde estimation de la pose de l'opérateur.

6. Système selon l'une quelconque des revendications précédentes, dans lequel le processeur est configuré pour identifier lorsque l'opérateur se trouve dans une zone de capture du capteur optique, et pour déclencher le capteur optique afin d'obtenir les données indicatives de la seconde estimation de la pose lorsqu'il a été identifié que l'opérateur se trouve dans la zone de capture.

7. Système selon l'une quelconque des revendications précédentes, comprenant en outre un dispositif de réalité augmentée, dans lequel le processeur est configuré, en réponse à la détermination que l'opérateur porte l'article vestimentaire de manière incorrecte, pour fournir un guidage à l'opérateur via le dispositif de réalité augmentée.

8. Système selon l'une quelconque des revendications précédentes, dans lequel le processeur est configuré, en réponse à la détermination que l'opérateur porte l'article vestimentaire de manière incorrecte, pour détecter une action dangereuse effectuée par l'opérateur en utilisant une base de données d'actions dangereuses pour détecter l'action humaine dangereuse.

9. Procédé mis en œuvre par ordinateur (800) pour effectuer une estimation de pose humaine, le procédé comprenant les étapes consistant à :
recevoir (S304), en provenance d'un ou plusieurs capteurs fixés à un vêtement, des données indicatives d'une première estimation d'une pose d'un opérateur portant l'article vestimentaire ;
recevoir (S308), à partir d'un capteur optique, des données indicatives d'une seconde estimation de la pose de l'opérateur ;
calculer (S312) une différence entre la première estimation de la pose et la seconde estimation de la pose ;
déterminer, sur la base de la différence calculée, si l'opérateur porte l'article vestimentaire de manière incorrecte ; et
alerter l'opérateur lorsqu'il est déterminé que l'opérateur porte l'article vestimentaire de manière incorrecte ;
dans lequel la première estimation et la seconde estimation incluent chacune respectivement des données définissant une position et une orientation d'une ou plusieurs articulations du corps de l'opérateur ;
dans lequel le calcul de la différence inclut le calcul d'une distance (S312) entre une articulation telle que définie dans la première articulation et la même articulation telle que définie dans la seconde estimation ; et
dans lequel le processeur est configuré pour déterminer que l'opérateur porte l'article vestimentaire de manière incorrecte en déterminant (S314) une similitude entre la distance calculée pour chaque articulation et une distance stockée dans une base de données d'erreurs, et dans lequel le processeur détermine que l'opérateur porte l'article vestimentaire de manière incorrecte lorsque la similitude déterminée (S316) est supérieure à un seuil.

10. Support de stockage non transitoire lisible par ordinateur contenant des instructions exécutables par machine qui, lorsqu'elles sont exécutées sur un processeur, amènent le processeur à exécuter le procédé selon la revendication 9.

11. Ordinateur comprenant un processeur et une mémoire, la mémoire contenant des instructions exécutables par machine qui, lorsqu'elles sont exécutées sur le processeur, amènent le processeur à :
recevoir, en provenance d'un ou plusieurs capteurs fixés à un vêtement, des données indicatives d'une première estimation d'une pose d'un opérateur portant l'article vestimentaire :
recevoir, en provenance d'un capteur optique, des données indicatives d'une seconde estimation de la pose de l'opérateur ;
calculer une différence entre la première estimation de la pose et la seconde estimation de la pose ;
déterminer, sur la base de la différence calculée, si l'opérateur porte l'article vestimentaire de manière incorrecte ; et
alerter l'opérateur lorsqu'il est déterminé que l'opérateur porte l'article vestimentaire de manière incorrecte ;
dans lequel la première estimation et la seconde estimation incluent chacune respectivement des données définissant une position et une orientation d'une ou plusieurs articulations du corps de l'opérateur ;
dans lequel le calcul de la différence inclut le calcul d'une distance entre une articulation telle que définie dans la première estimation et la même articulation telle que définie dans la seconde estimation ; et
dans lequel le processeur est configuré pour déterminer que l'opérateur porte l'article vestimentaire de manière incorrecte en déterminant une similitude entre la distance calculée pour chaque articulation et une distance stockée dans une base de données d'erreurs, et dans lequel le processeur détermine que l'opérateur porte l'article vestimentaire de manière incorrecte lorsque la similitude déterminée est supérieure à un seuil.
